(19) Europäisches Patentamt
European Patent Office
Office européen des brevets

(11) **EP 4 317 170 A1**

(12) **EUROPEAN PATENT APPLICATION**
published in accordance with Art. 153(4) EPC

(43) Date of publication:
**07.02.2024 Bulletin 2024/06**

(21) Application number: **22779648.9**

(22) Date of filing: **21.02.2022**

(51) International Patent Classification (IPC):
*C07K 1/14* $^{(2006.01)}$     *C12Q 1/68* $^{(2018.01)}$
*G01N 21/65* $^{(2006.01)}$     *G01N 30/88* $^{(2006.01)}$

(52) Cooperative Patent Classification (CPC):
**G01N 30/88; C07K 1/14; C12Q 1/68; G01N 21/65;**
G01N 30/34; G01N 30/96; G01N 2030/8818;
G01N 2030/8831

(86) International application number:
**PCT/JP2022/007006**

(87) International publication number:
**WO 2022/209422 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2021 JP 2021057497**

(71) Applicant: FUJIFILM Corporation
Tokyo 106-8620 (JP)

(72) Inventors:
• **NAKAMURA, Naoki**
  **Ashigarakami-gun, Kanagawa 258-8577 (JP)**
• **SUGITA, Yui**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR ESTIMATING PURIFIED STATE**

(57)     A method for estimating a purified state includes quantifying a component that is included in a treatment liquid obtained by performing a purification treatment on a liquid including a specific protein and impurities other than the protein. The method for estimating a purified state includes acquiring an estimated value of a concentration of the impurities on the basis of spectral data indicating an intensity of electromagnetic waves, which have been emitted to the treatment liquid and have been subjected to an action of the treatment liquid, for each wave number or wavelength. The concentration of the impurities included in the treatment liquid is equal to or less than 20 mg/mL, and a weight ratio of the impurities to a mixture including the protein and the impurities is equal to or less than 15%.

FIG. 4

50

| No. | SPECTRAL DATA | | | | | STATE DATA CONCENTRATION OF IMPURITIES [g/L] |
|---|---|---|---|---|---|---|
| | 500 cm$^{-1}$ | 501 cm$^{-1}$ | 502 cm$^{-1}$ | ⋯ | 3000 cm$^{-1}$ | |
| 1 | 0.21 | 0.51 | 0.14 | ⋯ | 0.32 | 0.23 |
| 2 | 0.11 | 0.81 | 0.32 | ⋯ | 0.96 | 0.34 |
| ⋮ | ⋮ | ⋮ | ⋮ | ⋮ | ⋮ | ⋮ |

EP 4 317 170 A1

**Description**

**BACKGROUND OF THE INVENTION**

1. Field of the Invention

The technology of the present disclosure relates to a method for estimating a purified state in a case in which a purification treatment is performed on a liquid including a specific protein.

2. Description of the Related Art

The following technique is known as a technique related to purification of proteins such as antibodies produced by cells. For example, US2020/0062802A discloses a technique that quantifies a purification intermediate of a protein which is being produced using in-line Raman spectroscopy.

**SUMMARY OF THE INVENTION**

In production of biopharmaceuticals, a protein, such as an antibody, which is a biopharmaceutical drug substance produced from cultured cells, is purified and formulated. In a protein purification step, for example, a purification treatment is performed by a plurality of different chromatography methods, such as cation chromatography, anion chromatography, immunoaffinity chromatography, and gel filtration chromatography, to increase the purity of a target protein stepwise. It is preferable to monitor the purified state in order to verify whether or not the purification treatment is appropriately performed in each step. In particular, it is important to quantify impurities separated from the target protein in each step. The reason is that, in a case in which impurities other than the target protein are mixed in the medicine, the impurities are likely to affect the efficacy of the medicine even though the amount of impurities is very small. In the purification step, the purity of the target protein is increased stepwise, and the amount of impurities included in the treatment liquid treated in each step is very small. Therefore, it is not easy to quantify the impurities. US2020/0062802A discloses a technique that quantifies the purification intermediate of the protein which is being produced, but does not disclose the quantification of impurities.

The technology of the present disclosure has been made in view of the above-described points, and an object of the technology of the present disclosure is to provide a method for estimating a purified state that can estimate the concentration of impurities with high accuracy even in a case in which the amount of impurities other than a protein, which is included in a treatment liquid subjected to a protein purification treatment, is very small.

According to the technology of the present disclosure, there is provided a method for estimating a purified state. The method comprises: quantifying a component that is included in a treatment liquid obtained by performing a purification treatment on a liquid including a specific protein and impurities other than the protein; and acquiring an estimated value of a concentration of the impurities on the basis of spectral data indicating an intensity of electromagnetic waves, which have been emitted to the treatment liquid and have been subjected to an action of the treatment liquid, for each wave number or wavelength. The concentration of the impurities included in the treatment liquid may be equal to or less than 20 mg/mL, and a weight ratio of the impurities to a mixture including the protein and the impurities may be equal to or less than 15%.

According to the technology of the present disclosure, there is provided a method for estimating a purified state. The method comprises: quantifying a component that is included in a treatment liquid obtained by performing a purification treatment on a liquid including a specific protein and impurities other than the protein; and acquiring an estimated value of a concentration of an immature sugar chain that has a structure similar to that of the protein on the basis of spectral data indicating an intensity of electromagnetic waves, which have been emitted to the treatment liquid and have been subjected to an action of the treatment liquid, for each wave number or wavelength.

The method for estimating a purified state according to the technology of the present disclosure may further comprise acquiring an estimated value of a concentration of the protein included in the treatment liquid on the basis of the spectral data. The specific protein may be produced from a cultured cell. The impurities may include DNA of a cell producing a specific antibody, an aggregate of the protein, a decomposition product of the protein, and a host cell protein. The purification treatment may include a component separation method using chromatography. A determination coefficient indicating a degree of match of the estimated value of the concentration of the impurities with a measured value may be equal to or greater than 0.9. A root mean squared error indicating a degree of deviation of the estimated value of the concentration of the impurities from a measured value may be equal to or less than 1.2.

The estimation method according to the technology of the present disclosure may further comprise: constructing a soft sensor, which receives the spectral data as an input and outputs state data indicating a purified state of the liquid including the protein and the impurities, with machine learning using a plurality of combinations of the state data and the spectral data as training data; and inputting the spectral data acquired for the treatment liquid to the soft sensor and acquiring the state data output from the soft sensor. The state data may include the estimated value of the concentration of the impurities included in the treatment liquid.

The method for estimating a purified state according to the technology of the present disclosure may further comprise: performing preprocessing on the spectral data; and constructing the soft sensor with machine learning using a plurality of combinations of processed data obtained by the preprocessing and the state data as training data. The preprocessing may include a process of selecting, from spectral intensity values for each wave number or wavelength included in the spectral data, a spectral intensity value used as the training data. Among the intensities for each wave number or wavelength included in the spectral data, the number of intensities selected to be used as the training data may be equal to or greater than 5 and less than 1000. The selection may be performed by sparse modeling. The preprocessing may include specifying high-correlation spectral data having a relatively high correlation with the state data among the spectral data as the processed data. The preprocessing may include a baseline correction of the spectral data.

The spectral data may be data indicating a spectrum of scattered light of light emitted to the liquid including the protein and the impurities. The state data may include an estimated value of a concentration of the protein included in the treatment liquid.

According to the technology of the present disclosure, there is provided a method for estimating a purified state that can estimate the concentration of impurities with high accuracy even in a case in which the amount of impurities other than a protein, which is included in a treatment liquid subjected to a protein purification treatment, is very small.

## BRIEF DESCRIPTION OF THE DRAWINGS

Fig. 1 is a diagram illustrating an example of an antibody purification step according to an embodiment of the technology of the present disclosure.

Fig. 2 is a diagram illustrating an example of a method for estimating a purified state according to the embodiment of the technology of the present disclosure.

Fig. 3 is a diagram illustrating an example of a method for acquiring spectral data.

Fig. 4 is a diagram illustrating an example of training data according to the embodiment of the technology of the present disclosure.

Fig. 5 is a diagram illustrating an example of the method for estimating the purified state according to the embodiment of the technology of the present disclosure.

Fig. 6 is a diagram illustrating an example of a hardware configuration of an information processing device according to the embodiment of the technology of the present disclosure.

Fig. 7 is a diagram illustrating an example of a structure of an estimation model according to the embodiment of the technology of the present disclosure.

Fig. 8 is an example of a functional block diagram illustrating an example of a functional configuration of the information processing device in a learning phase according to the embodiment of the technology of the present disclosure.

Fig. 9 is a flowchart illustrating an example of a flow of a soft sensor construction process according to the embodiment of the technology of the present disclosure.

Fig. 10 is an example of a functional block diagram illustrating an example of a functional configuration of the information processing device in an operation phase according to the embodiment of the technology of the present disclosure.

Fig. 11 is a flowchart illustrating an example of a flow of an estimation process according to the embodiment of the technology of the present disclosure.

Fig. 12A is a graph illustrating a relationship between an estimated value and a measured value of a concentration of impurities.

Fig. 12B is a graph illustrating the relationship between the estimated value and the measured value of the concentration of the impurities.

Fig. 12C is a graph illustrating the relationship between the estimated value and the measured value of the concentration of the impurities.

Fig. 13A is a graph illustrating a relationship between an estimated value and a measured value of a concentration of an antibody.

Fig. 13B is a graph illustrating the relationship between the estimated value and the measured value of the concentration of the antibody.

Fig. 13C is a graph illustrating the relationship between the estimated value and the measured value of the concentration of the antibody.

Fig. 14 is a graph illustrating a relationship between an estimated value and a measured value of a concentration of an immature sugar chain.

**DESCRIPTION OF THE PREFERRED EMBODIMENTS**

Hereinafter, an example of an embodiment of the technology of the present disclosure will be described with reference to the drawings. In addition, in each of the drawings, the same or equivalent components and portions are denoted by the same reference numerals, and the repeated description thereof will be omitted as appropriate.

A method for estimating a purified state according to an embodiment of the technology of the present disclosure includes quantifying a component that is included in a treatment liquid obtained by performing a purification treatment on a liquid including a specific protein and impurities other than the protein. More specifically, the method includes acquiring an estimated value of the concentration of the impurities included in the treatment liquid on the basis of spectral data indicating the intensity of electromagnetic waves, which have been emitted to the treatment liquid and have been subjected to the action of the treatment liquid, for each wave number or wavelength. The method for estimating the purified state according to the technology of the present disclosure is particularly effective in a case in which the concentration of the impurities included in the treatment liquid is equal to or less than 20 mg/mL and a weight ratio of the impurities to a mixture including the protein and the impurities is equal to or less than 15%. In addition, the method for estimating the purified state according to the technology of the present disclosure may include acquiring an estimated value of the concentration of the specific protein included in the treatment liquid.

The specific protein may be, for example, an immunoglobulin produced from cultured cells, that is, an antibody. The impurities include, for example, an immature sugar chain having a structure similar to that of the antibody, DNA of a cell, an aggregate of the antibody, a decomposition product of the antibody, and a host cell protein (HCP). The immature sugar chain having a structure similar to that of the antibody is likely to be formed, for example, in a case in which the amount of waste products in a culture solution increases or in a case in which oxygen concentration in the culture solution is insufficient for a culture period of antibody-producing cells. The decomposition product of the antibody is formed by the decomposition of the antibody by a degrading enzyme produced for the culture period. The aggregate of the antibody is likely to be formed, for example, in a case in which the concentration of the antibody produced from the cells is excessively high or in a case in which stress, such as heat, is applied. The DNA excreted from the cell means that a cell membrane of the cell has broken down, that is, the cell has become a dead cell. The host cell protein is a host cell-derived protein that is purified together with the antibody in the process of purifying the antibody. In a case in which the above-described impurities are mixed in a medicine using the antibody produced from cells, the impurities are likely to affect the efficacy of the medicine even though the amount of impurities is very small. Therefore, it is important to quantify the impurities in the treatment liquid obtained by the purification treatment for purifying the antibody.

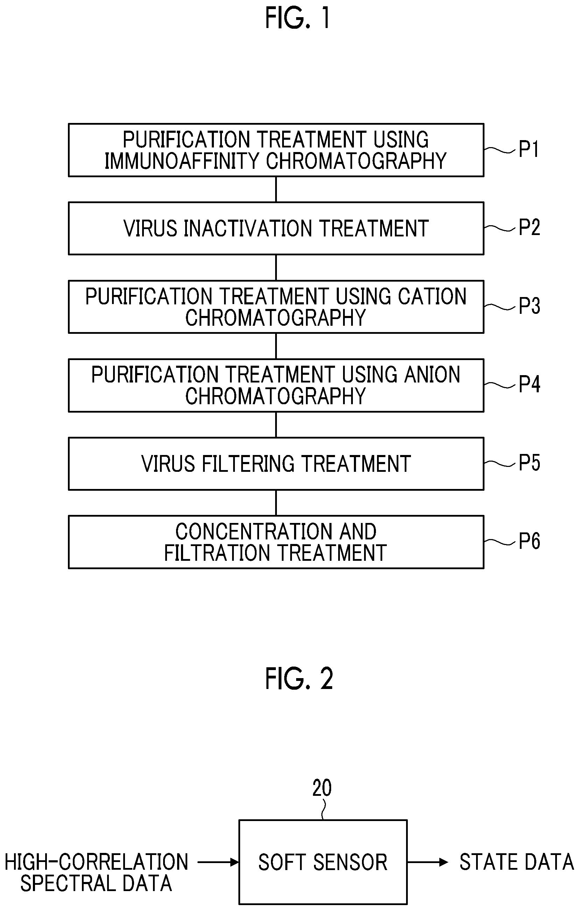

Fig. 1 is a diagram illustrating an example of an antibody purification step according to the embodiment of the technology of the present disclosure. As illustrated in Fig. 1, the antibody purification step includes a purification treatment P1 using immunoaffinity chromatography, a virus inactivation treatment P2, a purification treatment P3 using cation chromatography, a purification treatment P4 using anion chromatography, a virus filtering treatment P5, and a concentration and filtration treatment P6.

The purification treatment P1 using immunoaffinity chromatography is a treatment of extracting an antibody using a column in which a ligand, such as protein A having an affinity for the antibody, is immobilized on a carrier. The virus inactivation treatment P2 is a treatment of inactivating a virus included in the treatment liquid obtained by the purification treatment P1. The purification treatment P3 using cation chromatography is a treatment of extracting the antibody using a column having a cation exchanger as a stationary phase. The purification treatment P4 using anion chromatography is a treatment of extracting the antibody using a column having an anion exchanger as a stationary phase. The virus filtering treatment P5 is a treatment of removing the virus included in the treatment liquid obtained by each of the above-described treatments using a filter. The concentration and filtration treatment P6 is a concentration and filtration treatment using ultrafiltration (UF) and diafiltration (DF).

As described above, a plurality of treatments including a component separation method using a plurality of different types of chromatography are performed stepwise to eliminate the impurities stepwise and to increase the purity of the antibody stepwise. It is preferable to monitor the purified state in order to verify whether or not an appropriate treatment is performed in each step. The method for estimating the purified state according to the embodiment of the technology of the present disclosure can be used to estimate the purified state of each of the treatment liquids obtained in each of the treatments P1 to P6 illustrated in Fig. 1. In addition, preferably, the purified state is estimated for each of the treatments obtained in each of the treatments P1 to P6. In a case in which there is a next step, the estimated purified state can be used for the treatment in the next step to determine purification conditions. Preferably, it is possible to perform the purification treatment while estimating the concentration of the antibody, the concentration of the impurities, and the immature sugar chain in the treatment P1. Hereinafter, the details of the method for estimating the purified state according to the embodiment of the technology of the present disclosure will be described.

The method for estimating the purified state according to the embodiment of the technology of the present disclosure includes constructing a soft sensor, which receives spectral data as an input and outputs state data, with machine learning using a plurality of combinations of the state data and the spectral as training data. Here, the state data indicates a purified state of a liquid that includes a specific protein and impurities and that is to be subjected to the purification treatment, and the spectral data indicates the intensity of electromagnetic waves, which have been emitted to the treatment liquid obtained by the purification treatment and have been subjected to the action of the treatment liquid, for each wave number or wavelength. The method for estimating the purified state according to the embodiment of the technology of the present disclosure includes acquiring the state data that is output from the soft sensor in a case in which the spectral data acquired for the treatment liquid obtained by the purification treatment is input to the soft sensor. The state data includes an estimated value of the concentration of the impurities included in the treatment liquid.

In addition, the method for estimating the purified state according to the embodiment of the technology of the present disclosure includes: performing preprocessing on the spectral data; and constructing the soft sensor with machine learning using, as training data, a plurality of combinations of processed data obtained by the preprocessing and the state data. Dimensional reduction methods, such as sparse modeling, principal component analysis (PCA), latent semantic analysis (singular value decomposition) (LSA (SVD)), linear discriminant analysis (LDA), independent component analysis (ICA), and partial least squares regression (PLS), are used as a preprocessing method. The preprocessing method may include a process of selecting a spectral intensity value used as the training data among the spectral intensity values for each wave number or wavelength included in the spectral data. In this case, the remaining spectral intensity values for each wave number or wavelength after the selection are the processed data. It is assumed that the spectral intensity values for each wave number or wavelength constituting the spectral data are enormous. The selection of the data used as the training data makes it possible to prevent a reduction in the accuracy of prediction caused by over-training with model data. The selection of the spectral data can be performed, for example, by sparse modeling. That is, the preprocessing performed on the spectral data may include a process of excluding data having a relatively low correlation with the state data among the spectral data, using sparse modeling, to specify high-correlation spectral data having a relatively high correlation with the state data in the spectral data as the processed data. Among the intensity data for each wave number or wavelength included in the spectral data, the number of intensity data items which are used as the training data and selected by the preprocessing is preferably equal to or greater than 5 and less than 1000, more preferably equal to or greater than 5 and equal to or less than 800, and further preferably equal to or greater than 5 and equal to or less than 500.

In this embodiment, the sparse modeling means that explanatory variables are selected (that is, some of the explanatory variables are excluded) for a regression model which uses the spectral intensity values for each wave number or wavelength included in the spectral data as the explanatory variables and uses the state data as objective variables. For example, lasso regression can be used as a sparse modeling method. The lasso regression is a method that selects the explanatory variables such that a cost function calculated by adding a penalty term to a root mean squared error (RMSE) is minimized. In this embodiment, the explanatory variables are selected by excluding low-correlation spectral data having a relatively low correlation with the state data among the spectral data. The penalty term may be determined by, for example, cross-validation represented by K-fold cross validation. In the following description, a case in which the preprocessing performed on the spectral data is a process of specifying high-correlation spectral data will be described as an example.

The liquid including a specific protein and impurities other than the protein can be produced by a known method such as a method that cultures cells having a gene encoded with the specific protein, performs a cell removal treatment on the resulting culture, and performs the purification treatment using chromatography. For example, the liquid can be produced by culturing CHO cells, into which an IgG1 antibody gene has been introduced, performing the cell removal treatment using a filtering treatment, and performing a purification treatment using chromatography with protein A. The ratio of the specific protein to the impurities other than the protein can be changed by changing purification conditions such as the pH and temperature of the protein A. In this embodiment, an aqueous sodium acetate solution was used as a buffer solution during purification. In protein purification, a phosphate-based or acetic acid-based buffer solution is mainly used. Since the wave numbers characteristic of these buffer solutions are known, it is possible to perform prediction regardless of the buffer solution by removing the wave numbers.

The technology of the present disclosure can be applied regardless of the type of protein. The difference between antibody species is the difference between amino acid sequences. Since this difference between the amino acid sequences does not appear in a spectral difference, it can be adapted regardless of the type of antibody.

The technology of the present disclosure can be applied to immature sugar chains regardless of the type of the immature sugar chain.

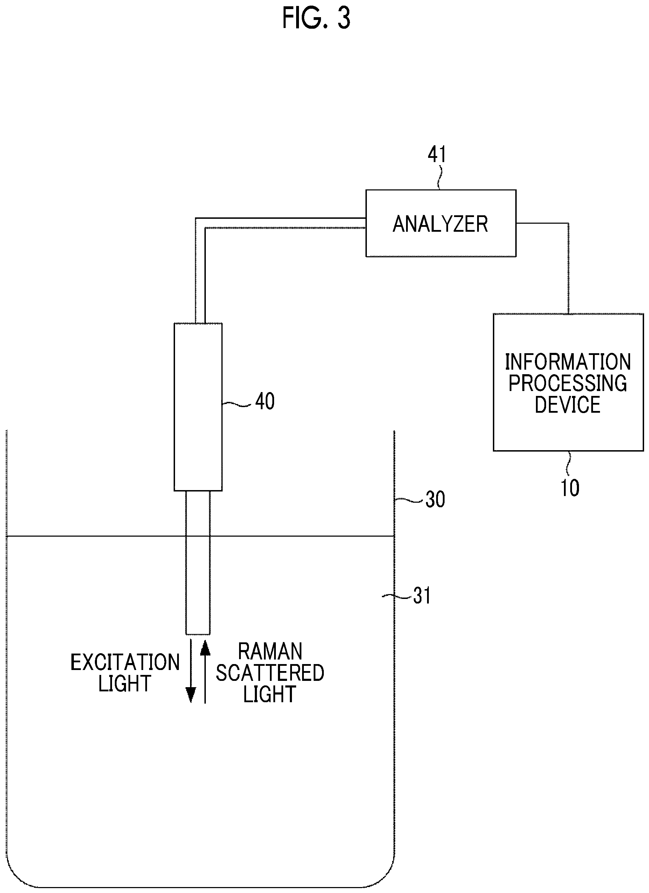

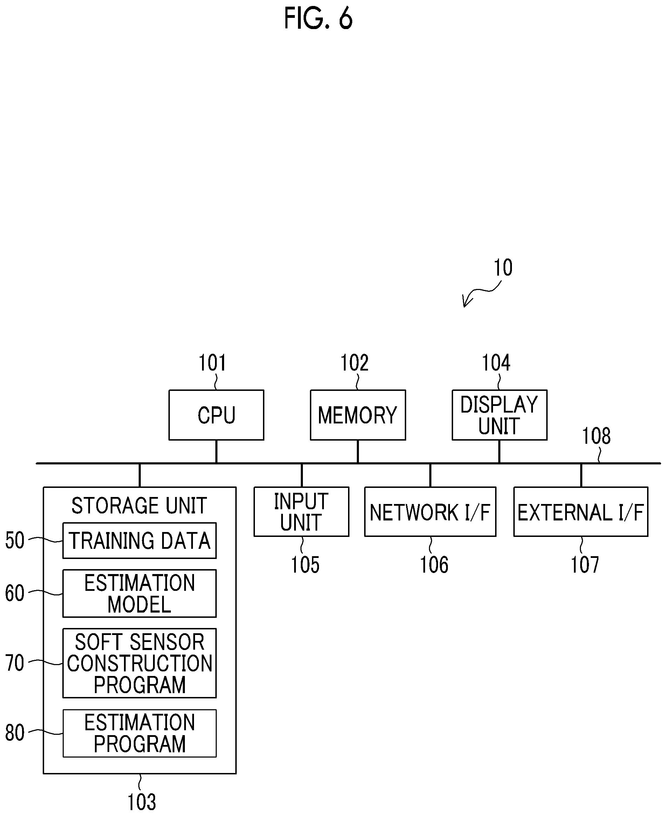

The method for estimating the purified state according to the embodiment of the technology of the present disclosure includes, for example, a step of acquiring state data output from a soft sensor 20 in a case in which, among the spectral data acquired for the treatment liquid obtained by any one of a plurality of treatments illustrated in Fig. 1 which are performed in the antibody purification step, high-correlation spectral data is input as the processed data to the soft sensor 20 as illustrated in Fig. 2. The soft sensor 20 implements a process of outputting the state data on the basis of the input high-correlation spectral data using software. The soft sensor 20 is constructed in an information processing device 10 (see Figs. 3 and 6) which will be described below.

In this embodiment, an analysis method using Raman spectroscopy is applied to the soft sensor 20. That is, spectral data of Raman scattered light is applied as the spectral data input to the soft sensor 20. The Raman spectroscopy is a spectroscopic method that evaluates a substance using the Raman scattered light. In a case in which a substance is irradiated with light, the light interacts with the substance to generate Raman scattered light having a wavelength different from that of incident light. Since a difference in wavelength between the incident light and the Raman scattered light corresponds to the molecular vibration energy of the substance, the Raman scattered light having a different wavelength (wave number) can be obtained between substances having different molecular structures. In addition, it is possible to estimate various physical properties, such as stress, temperature, electrical characteristics, orientation, and crystallinity, using the Raman scattered light. Of a Stokes line and an anti-Stokes line, the Stokes line is preferably used as the Raman scattered light. In this embodiment, Raman spectra were collected under the conditions of a laser output of 500 mW, a measurement wavelength of 785 nm, and a laser irradiation time of 1 second.

Fig. 3 is a diagram illustrating an example of a method for acquiring spectral data for a treatment liquid 31 obtained by any of the treatments P1 to P6 illustrated in Fig. 1. The spectral data can be acquired using a known probe 40 and a known analyzer 41 for Raman spectroscopic analysis. As illustrated in Fig. 3, the tip of the probe 40 is immersed in the treatment liquid 31 that is contained in a container 30. The treatment liquid 31 is irradiated with excitation light emitted from a light emitting unit (not illustrated) that is provided at the tip of the probe 40. The Raman scattered light generated by the interaction between the excitation light and the treatment liquid 31 is received by a light receiving unit (not illustrated) that is provided at the tip of the probe 40. The acquired Raman scattered light is decomposed for each wave number (the reciprocal of the wavelength) by the analyzer 41, and spectral data which is a spectral intensity value for each wave number is generated. In addition, the spectral data may be a spectral intensity value for each wavelength. The spectral data is supplied to the information processing device 10.

The state data output from the soft sensor 20 is data that indicates the purified state and is correlated with the spectral data. The state data includes an estimated value of the concentration of impurities included in the treatment liquid 31. The state data may include an estimated value of the concentration of an antibody included in the treatment liquid 31. It is not easy to monitor the state data in line using actual measurement. The use of the soft sensor 20 makes it possible to acquire the state data in line on the basis of the spectral data which is relatively easy to monitor in line using actual measurement.

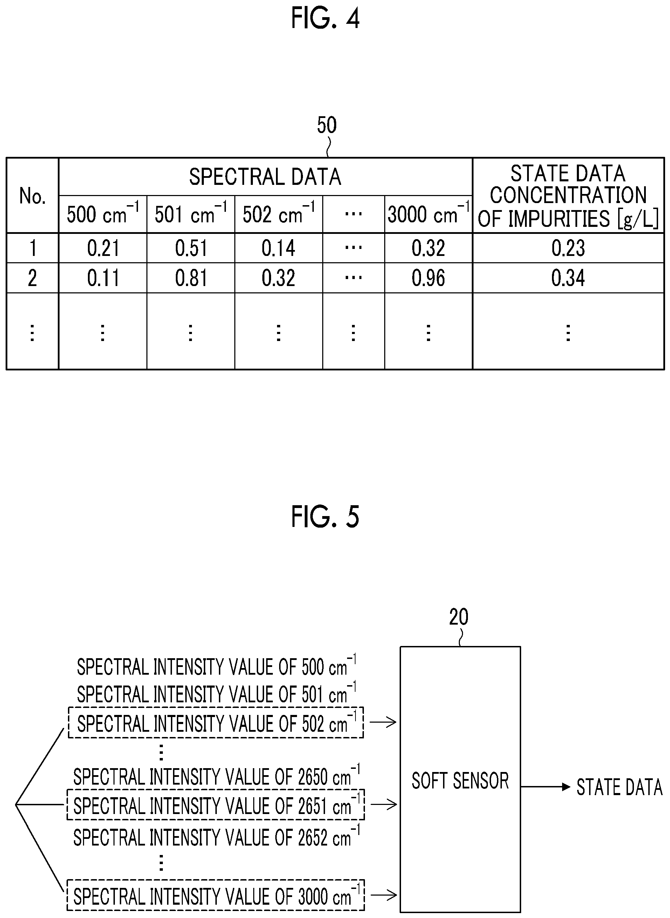

The soft sensor 20 is constructed by machine learning using a plurality of combinations of the spectral data and the state data as the training data. Fig. 4 is a diagram illustrating an example of training data 50. The training data 50 is acquired, for example, in a stage of process development in which purification treatment conditions are examined. Training spectral data is acquired from, for example, a treatment liquid obtained by changing various purification conditions. The purification conditions include, for example, a flow rate in a case in which the liquid to be purified is injected into the column, the amount of buffer used in a case in which the antibody is eluted from the column, and a composition of the buffer.

Training state data can be acquired by actually measuring the treatment liquid, from which the training spectral data has been acquired, using a sampling method according to the related art. For example, in a case in which the concentration of the impurities included in the treatment liquid is acquired as the training state data, it can be acquired by a method, such as high performance liquid chromatography (HPLC), for each type of impurities. The training data is acquired for each purification condition, and the training spectral data and the training state data under each condition are associated with each other.

Here, the analyzer 41 outputs, as the spectral data, the spectral intensity value in the range of, for example, a wave number of 500 cm$^{-1}$ to 3000 cm$^{-1}$ at an interval of 1 cm$^{-1}$. Therefore, the number of spectral data items acquired is enormous. In a case in which all of the spectral data is used as the training data, a learning load becomes excessive, and a high-performance processor is required in order to perform machine learning. Further, in some cases, the spectral intensity values of the Raman scattered light constituting the spectral data include a spectral intensity value for a wave number having a low correlation with the state data to be monitored. For example, it is considered that the spectral intensity value for a specific wave number value of the Raman scattered light has a low correlation with the concentration of the impurities. In a case in which the soft sensor 20 is constructed by machine learning using spectral data including the spectral intensity value of the wave number having a low correlation with the state data to be monitored as the training data, there is a concern that the accuracy of the output value of the soft sensor 20 will be reduced.

Therefore, in this embodiment, as the preprocessing on the spectral data, among the spectral data output from the analyzer 41, the spectral intensity value of the wave number having a relatively high correlation with the state data to be monitored is specified as the high-correlation spectral data. Then, in a learning phase which is a stage of constructing the soft sensor 20 using machine learning, the soft sensor 20 is constructed by machine learning using a plurality of combinations of the high-correlation spectral data and the state data as the training data. On the other hand, in an operation phase in which the constructed soft sensor 20 is operated to acquire state data for the treatment liquid obtained by the purification treatment, as illustrated in Fig. 5, among the spectral data acquired for the treatment liquid obtained by the purification treatment, high-correlation spectral data having a relatively high correlation with the state data to be monitored is input to the soft sensor 20, and the state data output from the soft sensor 20 is acquired. The information processing device 10 constructs the soft sensor 20 and acquires the state data using the soft sensor 20.

The color of the treatment liquid for which the spectral data is to be acquired changes depending on, for example, the amount of impurities included in the treatment liquid, the type of the antibody, and the type of the antibody-producing cell. In addition, for example, fluctuations in the external environment, such as temperature, humidity, and vibration, in the acquisition of the spectral data, and the output of the excitation light emitted to the treatment liquid are disturbances to the spectral data. In some cases, these factors cause fluctuations in the baseline of the spectral data. The fluctuations in the baseline cause a reduction in the accuracy of the output value of the soft sensor 20. Therefore, in this embodiment, the correction of the baseline of the spectral data is further performed as the preprocessing on the spectral data. The correction of the baseline means removing the fluctuations caused by the disturbance at the baseline of the spectral data. The correction of the baseline may be performed, for example, by performing differential processing on a spectral waveform. In addition, for example, the correction of the baseline may be performed by removing a baseline calculated by polynomial fitting from the spectral waveform.

Fig. 6 is a diagram illustrating an example of a hardware configuration of the information processing device 10. The information processing device 10 includes a central processing unit (CPU) 101, a memory 102 as a temporary storage area, and a non-volatile storage unit 103. Further, the information processing device 10 includes a display unit 104, such as a liquid crystal display, an input unit 105, such as a keyboard or a mouse, a network interface (I/F) 106 connected to a network, and an external I/F 107 to which the analyzer 41 is connected. The CPU 101, the memory 102, the storage unit 103, the display unit 104, the input unit 105, the network I/F 106, and the external I/F 107 are connected to a bus 108.

The storage unit 103 is implemented by a storage medium, such as a hard disk drive (HDD), a solid state drive (SSD), or a flash memory. The training data 50, an estimation model 60, a soft sensor construction program 70, and an estimation program 80 are stored in the storage unit 103. As illustrated in Fig. 4, the training data 50 is a plurality of combinations of the spectral data and the state data.

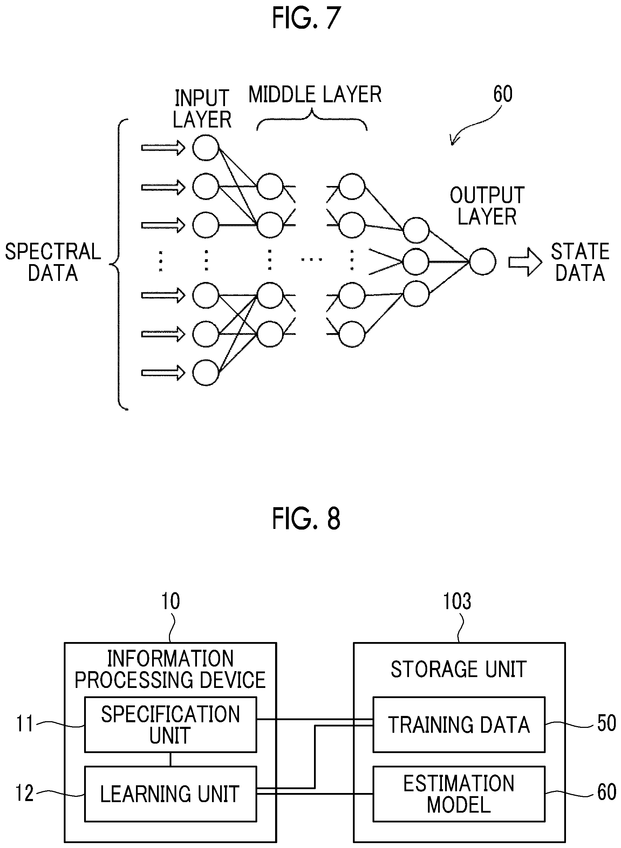

Fig. 7 is a diagram illustrating an example of a structure of the estimation model 60. The estimation model 60 is a neural network including an input layer, a plurality of middle layers, and an output layer. The spectral intensity value for each wave number of the Raman scattered light, that is, the spectral data is input to the input layer of the estimation model 60. State data corresponding to the spectral data input to the input layer is output from the output layer of the estimation model 60.

In the learning phase, the CPU 101 reads the soft sensor construction program 70 from the storage unit 103, expands the soft sensor construction program 70 in the memory 102, and executes the soft sensor construction program 70. In the operation phase, the CPU 101 reads the estimation program 80 from the storage unit 103, expands the estimation program 80 in the memory 102, and executes the estimation program 80. In addition, a server computer is given as an example of the information processing device 10. The CPU 101 is an example of a processor according to the technology of the present disclosure.

Fig. 8 is an example of a functional block diagram illustrating an example of a functional configuration of the information processing device 10 in the learning phase. In the learning phase, the information processing device 10 is configured to include a specification unit 11 and a learning unit 12. It is assumed that the training data 50 and the estimation model 60 are stored in the storage unit 103.

The specification unit 11 performs regression analysis on the training data 50, using lasso regression which is an example of the sparse modeling, to specify the spectral intensity value of the wave number having a relatively high correlation with the state data as the high-correlation spectral data among the spectral data included in the training data 50. Specifically, the specification unit 11 performs the following process. The specification unit 11 performs a process of thinning out the spectral intensity value of the wave number, which has been determined randomly, on the spectral data included in the training data 50 and generates a regression model (regression expression) indicating the relationship between the thinned-out spectral data and the corresponding state data. The specification unit 11 derives a cost function obtained by adding a penalty term to a root mean squared error (RMSE) for the generated regression model. The specification unit 11 repeatedly performs each of the above-mentioned processes a predetermined number of times to generate the regression model for each of a plurality of spectral data items having different wave numbers to be thinned out and to derive the above-mentioned cost function for each regression model. The specification unit 11 specifies the smallest number of spectral intensity values that can minimize the above-mentioned cost function as the high-correlation spectral data in a predetermined number of repeated calculation operations.

The learning unit 12 trains the estimation model 60 with machine learning using a combination of the high-correlation spectral data specified by the specification unit 11 and the corresponding state data in the training data 50 as teacher data. Therefore, the soft sensor 20 that receives the high-correlation spectral data as an input and outputs the state data is constructed.

The learning unit 12 trains the estimation model 60 using the training data 50 according to a back-propagation method which is an example of machine learning. Specifically, the learning unit 12 extracts the high-correlation spectral data specified by the specification unit 11 from the training spectral data included in the training data 50. The learning unit 12 inputs the extracted high-correlation spectral data to the estimation model 60 and acquires the state data output from the estimation model 60. The learning unit 12 trains the estimation model 60 such that a difference between a score indicated by the acquired state data and a score indicated by the training state data corresponding to the high-correlation spectral data included in the training data 50 is minimized. The learning unit 12 performs a process of training the estimation model 60 using a combination of all or some of the high-correlation spectral data and the state data included in the training data 50. Further, in addition to the back-propagation method, random forest, linear regression, non-linear regression (Sapport vector machine (SVM) and Basian regression), logistic regression, and the like are given as examples of the machine learning method. However, the back-propagation method is preferable.

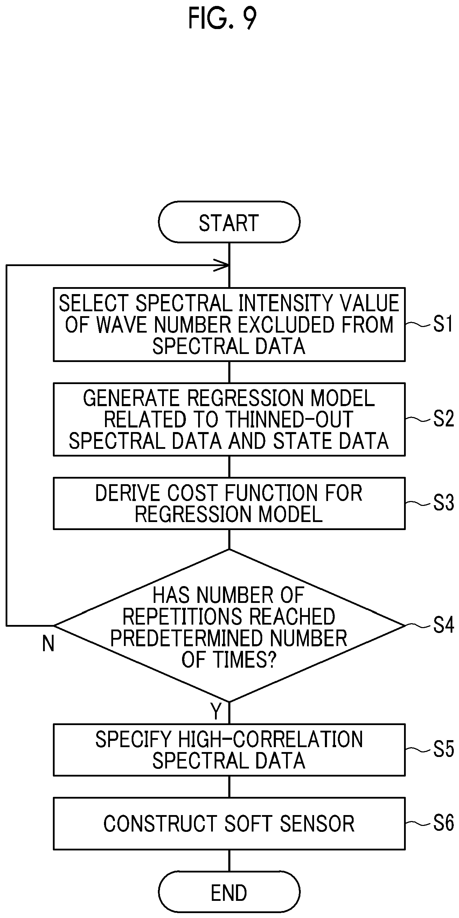

Fig. 9 is a flowchart illustrating an example of a flow of a soft sensor construction process performed by the execution of the soft sensor construction program 70 by the CPU 101 in the learning phase. The soft sensor construction program 70 is executed, for example, in a case in which an instruction to perform the soft sensor construction process is input by the user through the input unit 105.

In Step S1, the specification unit 11 randomly selects the spectral intensity value of the wave number to be excluded from the spectral data included in the training data 50 stored in the storage unit 103. That is, the specification unit 11 performs a process of thinning out the spectral intensity values for some wave numbers among the spectral intensity values acquired at a wave number interval of 1 cm$^{-1}$. The number of wave numbers to be excluded may be predetermined or randomly determined. It is preferable that a predetermined number of wave numbers are excluded.

In Step S2, the specification unit 11 generates a regression model (regression expression) indicating the relationship between spectral data (that is, thinned-out spectral data) composed of the spectral intensity values of wave numbers other than the wave numbers to be excluded, which have been selected in Step S1, and the corresponding state data. Specifically, a regression model that uses the thinned-out spectral data as an explanatory variable and uses the corresponding state data as an objective variable is estimated by a statistical method. The regression model may be a linear model or a non-linear model.

In Step S3, the specification unit 11 derives a cost function for the regression model generated in Step S2. The cost function is used as an index value indicating the accuracy of the regression model.

In Step S4, the specification unit 11 determines whether or not the number of repetitions of the processes from Step S1 to Step S3 has reached a predetermined number of times. The specification unit 11 repeatedly performs the processes from Step S1 to Step S3 until the number of repetitions reaches a predetermined number of times. Therefore, the regression model is generated for each of a plurality of thinned-out spectral data items having different wave numbers to be excluded, and the cost function is derived for each of the generated regression models.

In Step S5, the specification unit 11 specifies the thinned-out spectral data used to generate the regression model having the minimum cost function as the high-correlation spectral data. The spectral data used to generate the regression model having the minimum cost function is composed of the spectral intensity value of the wave number having a relatively high correlation with the state data. In this way, the specification unit 11 specifies the spectral data composed of the spectral intensity values of the wave numbers having a relatively high correlation with the state data as the high-correlation spectral data, using the regression analysis.

In Step S6, the learning unit 12 extracts the high-correlation spectral data specified in Step S5 from the spectral data included in the training data 50 stored in the storage unit 103 and trains the estimation model 60 with machine learning using a plurality of combinations of the extracted high-correlation spectral data and the corresponding state data as the teacher data. Specifically, the learning unit 12 inputs the high-correlation spectral data specified in Step S5 to the estimation model 60 and trains the estimation model 60 such that the difference between the score indicated by the state data output from the estimation model 60 and the score indicated by the training state data corresponding to the high-correlation spectral data included in the training data 50 is minimized. In this way, the soft sensor 20 is constructed.

The soft sensor 20 is constructed for each type of state data to be monitored. For example, in a case in which the estimated value of the concentration of the impurities included in the treatment liquid obtained by the purification treatment is output as the state data from the soft sensor 20, a spectral intensity value of a wave number having a high correlation with the concentration of the impurities among the spectral data is specified as the high-correlation spectral data. Then, the soft sensor 20 that outputs the estimated value of the concentration of the impurities on the basis of the high-correlation spectral data is constructed by machine learning using a plurality of combinations of the specified high-correlation spectral data and the state data indicating the concentration of the impurities acquired by actual measurement as the training data. Meanwhile, in a case in which the estimated value of the concentration of the antibody is output as the state data from the soft sensor 20, the spectral intensity value of the wave number having a high correlation with the concentration of the antibody among the spectral data is specified as the high-correlation spectral data. Then, the soft sensor 20 that outputs the estimated value of the concentration of the antibody on the basis of the high-correlation spectral data is constructed by machine learning using a plurality of combinations of the specified high-correlation spectral data and the state data indicating the concentration of the antibody acquired by actual measurement as the training data.

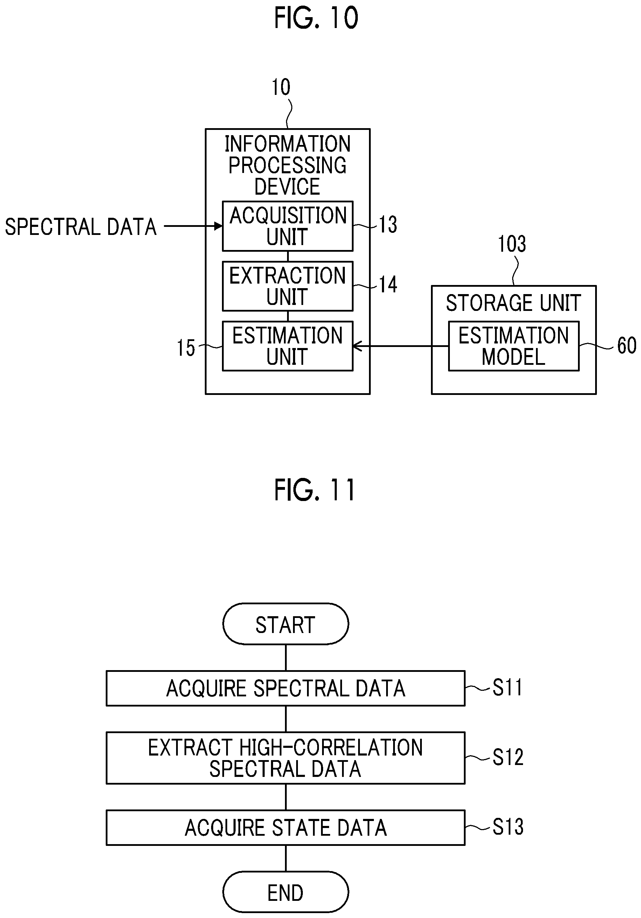

Fig. 10 is an example of a functional block diagram illustrating an example of a functional configuration of the information processing device 10 in the operation phase. In the operation phase, the information processing device 10 is configured to include an acquisition unit 13, an extraction unit 14, and an estimation unit 15. It is assumed that the storage unit 103 stores the trained estimation model 60 functioning as the soft sensor 20.

The method for estimating the purified state according to the embodiment of the technology of the present disclosure is applied, for example, to a case in which a component of the treatment liquid obtained by the purification treatment for extracting the antibody is quantified. As illustrated in Fig. 3, the spectral data is acquired for the treatment liquid 31 contained in the container 30 by the probe 40 and the analyzer 41.

The acquisition unit 13 acquires the spectral data output from the analyzer 41. The extraction unit 14 extracts the high-correlation spectral data specified by the specification unit 11, that is, the spectral intensity value of the wave number having a relatively high correlation with the state data to be monitored, among the spectral data acquired by the acquisition unit 13.

The estimation unit 15 reads the trained estimation model 60 functioning as the soft sensor 20 from the storage unit 103, inputs the high-correlation spectral data extracted by the extraction unit 14 to the estimation model 60, and acquires the state data output from the estimation model 60. The estimation unit 15 may perform control to display the acquired state data on the display unit 104. Further, the estimation unit 15 may store the acquired state data in the storage unit 103.

Fig. 11 is a flowchart illustrating an example of a flow of an estimation process performed by the execution of the estimation program 80 by the CPU 101 in the operation phase. The estimation program 80 is executed, for example, in a case in which an instruction to perform the estimation process is input by the user through the input unit 105.

In Step S11, the acquisition unit 13 acquires the spectral data output from the analyzer 41. In Step S12, the extraction unit 14 extracts the high-correlation spectral data specified by the specification unit 11, that is, the spectral intensity value of the wave number having a relatively high correlation with the state data to be monitored, among the spectral data acquired by the acquisition unit 13. In Step S13, the estimation unit 15 reads the trained estimation model 60 functioning as the soft sensor 20 from the storage unit 103, inputs the high-correlation spectral data extracted in Step S12 to the read estimation model 60, and acquires the state data output from the estimation model 60. The estimation unit 15 performs control to display the acquired state data on the display unit 104.

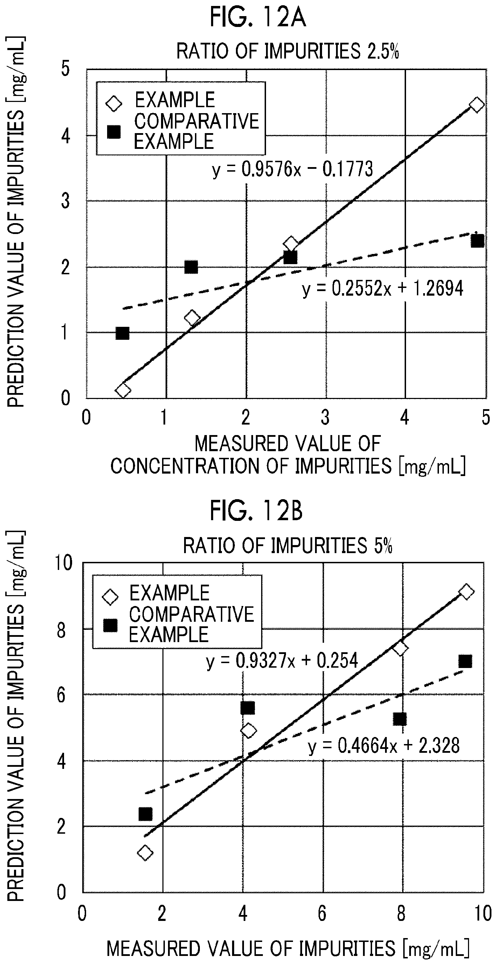

The estimated value of the concentration of the impurities included in the treatment liquid obtained by performing the purification treatment on the liquid including the antibody and the impurities was acquired by the soft sensor 20. Each of Figs. 12A to 12C is a graph illustrating the relationship between the estimated value of the concentration of the impurities acquired by the soft sensor 20 and the measured value of the concentration of the impurities acquired by sampling. Figs. 12A to 12C also illustrate, as a comparative example, the relationship between the estimated value of the concentration of the impurities acquired by analyzing the spectral data using PLS, which is one of the multivariate analysis methods, and the measured value. In Figs. 12A to 12C, the treatment liquid obtained by the treatment P1 was used as the liquid including the antibody and the impurities. In Figs. 12A to 12C, a case (Example) in which the estimated value of the concentration of the impurities is acquired by the soft sensor 20 is represented by a white diamond-shaped plot and a solid line, and a case (comparative example) in which the estimated value of the concentration of the impurities is acquired by PLS is represented by a black square-shaped plot and a dotted line.

A protein and impurities which are included in a liquid including the protein and the impurities other than the protein and a protein having an immature sugar chain can be actually measured by a known method. For example, the protein can be measured by subjecting the liquid to protein A chromatography. The impurities can be measured by performing a size exclusion chromatography treatment. A sugar chain liberation treatment can be performed on the immature sugar chain, the liberated sugar chain can be fluorescently labeled, an unreacted substance can be removed, and the concentration of the immature sugar chain can be measured by HPLC.

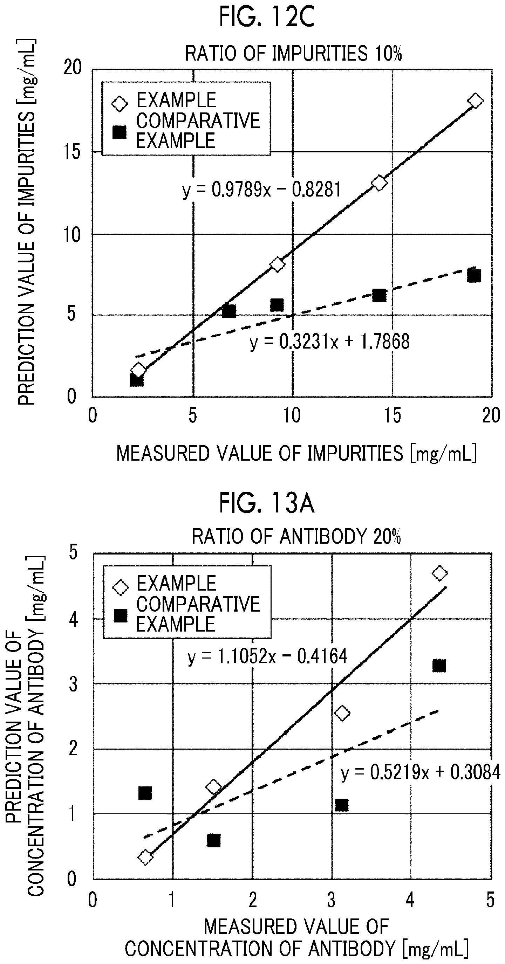

Fig. 12A illustrates a case in which the ratio of the impurities is 2.5%, Fig. 12B illustrates a case in which the ratio of the impurities is 5%, and Fig. 12C illustrates a case in which the ratio of the impurities is 10%. The ratio of the impurities is the weight ratio of the impurities to a mixture including the antibody and the impurities and is defined by the following Expression (1). In Expression (1), Rc is the ratio of the impurities, A is the weight of the antibody included in the treatment liquid, and C is the weight of the impurities included in the treatment liquid.

$$R_C = C/(A + C) \cdots (1)$$

The following Table 1 shows the results of calculating a determination coefficient ($R^2$) indicating the degree of match of each of the estimated value according to Example and the estimated value according to the comparative example with the measured value and a root mean squared error (RMSE) indicating the degree of deviation of each of the estimated values from the measured value.

[Table 1]

| Error Between Estimated Value and Measured Value of Concentration of Impurities | | | |
|---|---|---|---|
| Ratio of impurities | Evaluation index | Example | Comparative Example |
| 2.5% | Determination coefficient | 0.996 | 0.663 |
| | Root mean squared error | 0.301 | 1.357 |
| 5% | Determination coefficient | 0.973 | 0.750 |
| | Root mean squared error | 0.547 | 2.019 |
| 10% | Determination coefficient | 0.997 | 0.792 |
| | Root mean squared error | 1.100 | 6.627 |

As illustrated in Figs. 12A to 12C and Table 1, it was confirmed that the accuracy of the estimated value of the concentration of the impurities acquired by the soft sensor 20 was higher than the accuracy of the estimated value of the concentration of the impurities acquired by PLS. In particular, even in a case in which the concentration of the impurities was equal to or less than 5 mg/mL and the ratio of the impurities was 2.5%, the use of the soft sensor 20 made it possible to estimate the concentration of the impurities with extremely high accuracy.

The estimated value of the concentration of the antibody included in the treatment liquid obtained by performing the purification treatment on the liquid including the antibody and the impurities was acquired by the soft sensor 20. Each of Figs. 13A to 13C is a graph illustrating the relationship between the estimated value of the concentration of the antibody acquired by the soft sensor 20 and the measured value of the concentration of the antibody acquired by sampling. Figs. 13A to 13C also illustrate, as a comparative example, the relationship between the estimated value of the concentration of the antibody acquired by analyzing the spectral data using PLS, which is one of the multivariate analysis methods, and the measured value. In Figs. 13A to 13C, the treatment liquid obtained by the treatment P1 was used as the liquid including the antibody and the impurities. In Figs. 13A to 13C, a case (Example) in which the estimated value of the concentration of the antibody is acquired by the soft sensor 20 is represented by a white diamond-shaped plot and a solid line, and a case (comparative example) in which the estimated value of the concentration of the antibody is acquired by PLS is represented by a black square-shaped plot and a dotted line.

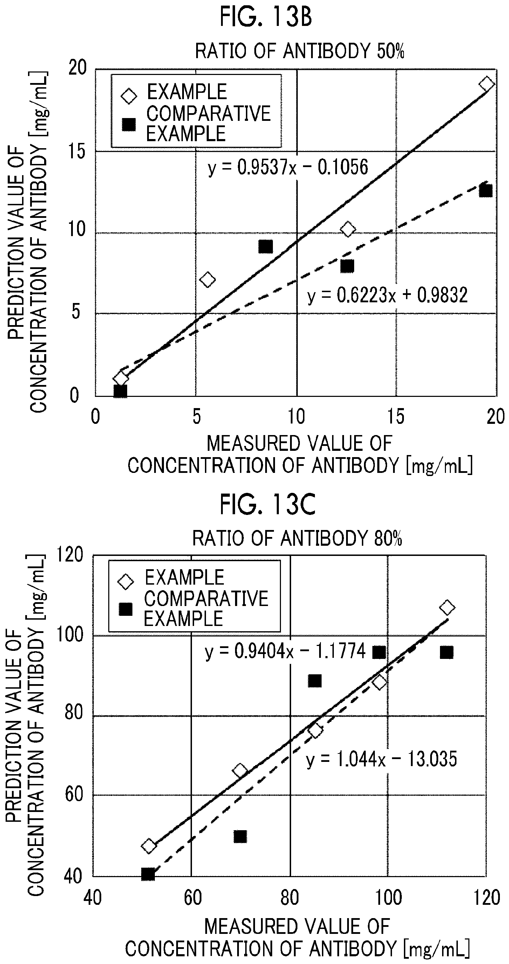

Fig. 13A illustrates a case in which the ratio of the antibody is 20%, Fig. 13B illustrates a case in which the ratio of the antibody is 50%, and Fig. 13C illustrates a case in which the ratio of the antibody is 80%. The ratio of the antibody is the weight ratio of the antibody to the mixture including the antibody and the impurities and is defined by the following Expression (2). In Expression (2), $R_A$ is the ratio of the antibody, A is the weight of the antibody included in the treatment liquid, and C is the weight of the impurities included in the treatment liquid.

$$R_A = A/(A + C) \cdots (2)$$

The following Table 2 shows the results of calculating a determination coefficient ($R^2$) indicating the degree of match of each of the estimated value according to Example and the estimated value according to the comparative example with the measured value and a root mean squared error (RMSE) indicating the degree of deviation of each of the estimated values from the measured value.

[Table 2]

| Error Between Estimated Value and Measured Value of Concentration of Antibody | | | |
|---|---|---|---|
| Ratio of antibody | Evaluation index | Example | Comparative Example |
| 20% | Determination coefficient | 0.969 | 0.549 |
| | Root mean squared error | 0.361 | 1.281 |
| 50% | Determination coefficient | 0.975 | 0.861 |
| | Root mean squared error | 1.210 | 4.228 |

(continued)

| Error Between Estimated Value and Measured Value of Concentration of Antibody | | | |
|---|---|---|---|
| Ratio of antibody | Evaluation index | Example | Comparative Example |
| 80% | Determination coefficient | 0.989 | 0.867 |
| | Root mean squared error | 6.634 | 12.812 |

As illustrated in Figs. 13A to 13C and Table 2, it was confirmed that the accuracy of the estimated value of the concentration of the antibody acquired by the soft sensor 20 was higher than the accuracy of the estimated value of the concentration of the antibody acquired by PLS. In particular, even in a case in which the concentration of the antibody was equal to or less than 5 mg/mL and the ratio of the antibody was 20%, the use of the soft sensor 20 made it possible to estimate the concentration of the antibody with extremely high accuracy.

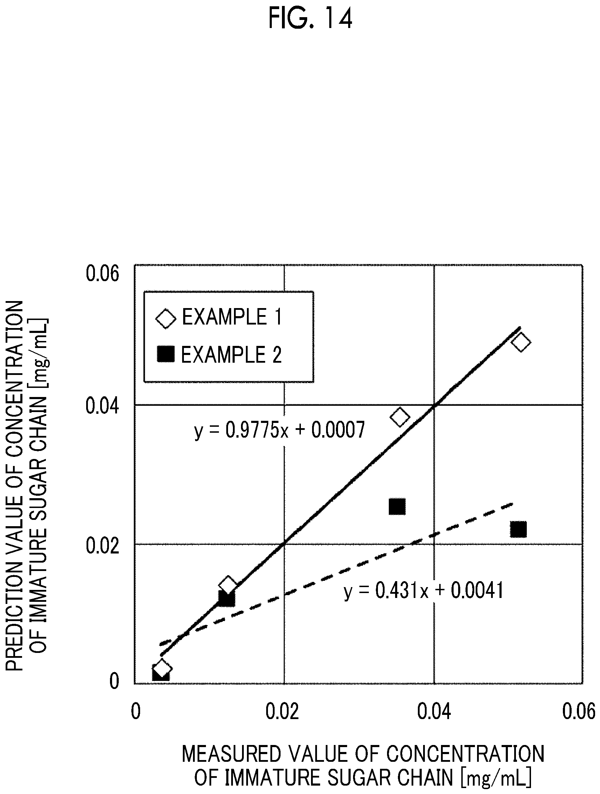

An estimated value of the concentration of an immature sugar chain, which is a kind of impurities included in a treatment liquid obtained by performing the purification treatment on a liquid including an antibody and impurities was acquired by the soft sensor 20. Fig. 14 is a graph illustrating the relationship (Example 1) between the estimated value of the concentration of the immature sugar chain acquired by the soft sensor 20 and the measured value of the concentration of the immature sugar chain acquired by sampling. Fig. 14 also illustrates the relationship (Example 2) between the estimated value of the concentration of the immature sugar chain acquired by analyzing the spectral data using PLS, which is one of the multivariate analysis methods, and the measured value. In Fig. 14, a case (Example 1) in which the estimated value of the concentration of the immature sugar chain is acquired by the soft sensor 20 is represented by a white diamond-shaped plot and a solid line, and a case (Example 2) in which the estimated value of the concentration of the antibody is acquired by PLS is represented by a black square-shaped plot and a dotted line.

The following Table 3 illustrates the results of calculating a determination coefficient ($R^2$) indicating the degree of match of each of the estimated value according to Example 1 and the estimated value according to Example 2 with the measured value and a root mean squared error (RMSE) indicating the degree of deviation of each of the estimated values from the measured value.

[Table 3]

| Error Between Estimated Value and Measured Value of Concentration of Immature Sugar Chain | | |
|---|---|---|
| Evaluation index | Example 1 | Example 2 |
| Determination coefficient | 0.985 | 0.779 |
| Root mean squared error | 0.0023 | 0.00157 |

As illustrated in Fig. 14C and Table 3, it was confirmed that the accuracy of the estimated value of the concentration of the immature sugar chain acquired by the soft sensor 20 was higher than the accuracy of the estimated value of the concentration of the immature sugar chain acquired by PLS.

As described above, according to the method for estimating the purified state according to the embodiment of the technology of the present disclosure, even in a case in which the amount of impurities other than the protein included in the treatment liquid, which has been subjected to the purification treatment for extracting a specific protein, is very small, it is possible to estimate the concentration of the impurities with high accuracy. For example, even in a case in which the concentration of the impurities included in the treatment liquid is equal to or less than 20 mg/mL and the ratio of the impurities is equal to or less than 15%, it is possible to estimate the concentration of the impurities with high accuracy.

In addition, since the in-line monitoring of the spectral data by actual measurement is relatively easy, it is possible to estimate the purified state in line. Further, it is possible to immediately acquire the estimation result of the purified state. Therefore, for example, the technology of the present disclosure can be applied to the purification treatment in the manufacture of medicines, which makes it possible to immediately (for example, within 10 seconds) respond to a case in which some abnormality occurs during the purification treatment. Furthermore, the technology of the present disclosure can be applied in the stage of process development in which the purification treatment conditions are examined, which makes it possible to evaluate the validity of the purification conditions in a short time.

Further, among the spectral data output from the analyzer 41, the high-correlation spectral data composed of the spectral intensity value of the wave number having a relatively high correlation with the state data to be monitored is used as the training data. Therefore, a learning load can be less than that in a case in which all of the spectral data output from the analyzer 41 is used as the training data. In addition, it is possible to improve the accuracy of the output value of the soft sensor 20.

In a case in which impurities are mixed in a medicine using the antibody produced from cells, the impurities are likely to affect the efficacy of the medicine even though the amount of impurities is very small. According to the method for estimating the purified state according to the embodiment of the technology of the present disclosure, it is possible to acquire the estimated value of the concentration of impurities. Therefore, the technology of the present disclosure can be applied to the purification treatment performed in the process of manufacturing medicines, which makes it possible to ensure the quality of the medicines.

Further, in this embodiment, the aspect in which the spectrum of Raman scattered light is used as the spectral data has been described as an example. However, the present disclosure is not limited to this aspect. For example, the absorption spectrum of infrared rays emitted to the treatment liquid subjected to the purification treatment may be used as the spectral data. In addition, a nuclear magnetic resonance spectrum may be used as the spectral data. It is preferable to use the spectrum of the Raman scattered light as the spectral data.

Further, in this embodiment, a case in which preprocessing is performed on the spectral data and the soft sensor is constructed by machine learning using a plurality of combinations of the processed data obtained by the preprocessing and the state data as the training data has been described as an example. However, in a case in which a reduction in the accuracy of the estimation model caused by the learning load and over-training does not cause a problem, spectral data that has not been subjected to the preprocessing may be used as the training data.

Furthermore, in this embodiment, a process for specifying the high-correlation spectral data having a relatively high correlation with the state data among the spectral data has been described as an example of the preprocessing. However, the present disclosure is not limited thereto. For example, a process that excludes the spectral intensity value of a predetermined wave number among the spectral data acquired by the analyzer 41 from the training data may be performed as the preprocessing. In addition, a process that groups the spectral data acquired by the analyzer 41 such that wave numbers close to each other belong to the same wave number group and calculates, for example, the average value, standard deviation, median value, maximum value, and minimum value of the intensity of scattered light for each wave number group may be performed as the preprocessing. In this case, the spectral intensity value for each wave number group is used as the training data. Further, a process that reduces the number of dimensions for the training data composed of a plurality of combinations of the spectral data indicating intensity for each wave number or each wavelength and the state data may be performed as the preprocessing.

An example of the utilization of the method for estimating the purified state according to the embodiment of the technology of the present disclosure will be described below. For example, in affinity chromatography and cation chromatography included in a step of manufacturing an antibody product or the like, the amount of a specific component adsorbed to a column is calculated in advance, and then a predetermined amount of liquid to be treated is introduced into the column. The method according to this embodiment is applied to the treatment liquid obtained by the purification treatment to estimate the amount of specific component. In a case in which the specific component is not adsorbed to the column and flows out of the column, it is possible to immediately detect this situation and to respond to this situation. For example, it is possible to reduce the introduction amount of the liquid to be treated into the column or to stop the introduction of the liquid to be treated into the column.

In addition, in the affinity chromatography and the cation exchange chromatography, impurities may be adsorbed to the column due to functional deterioration of the column, and the treatment liquid eluted from the column may not include the impurities or the amount of impurities may be less than usual. The method according to this embodiment can be applied to the treatment liquid eluted from the column to estimate the amount of impurities, which makes it possible to immediately detect this situation and to respond to this situation. For example, it is possible to replace the column.

In addition, in the cation exchange chromatography, a specific component is eluted from the column by applying a gradient at a salt concentration. The method according to this embodiment may be applied to the treatment liquid eluted from the column to estimate the amount of a specific component, and a gradient curve may be controlled according to the concentration of the specific component.

Furthermore, in the above-described embodiment, for example, the following various processors can be used as a hardware structure of processing units performing various processes such as the specification unit 11, the learning unit 12, the acquisition unit 13, the extraction unit 14, and the estimation unit 15. The various processors include, for example, a CPU which is a general-purpose processor executing software (program) to function as various processing units, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or a combination of two or more processors of the same type or different types (for example, a combination of a plurality of FPGAs or a combination of a CPU and an FPGA). In addition, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system on chip (SoC). As described above, various processing units are configured by using one or more of the various processors as a hardware structure. In addition, specifically, an electric circuit (circuitry) obtained by combining circuit elements, such as semiconductor elements, can be used as the hardware structure of the various processors.

Further, in the above-described embodiment, the aspect in which the soft sensor construction program 70 and the estimation program 80 are stored (installed) in the storage unit 103 in advance has been described. However, the present disclosure is not limited thereto. The soft sensor construction program 70 and the estimation program 80 may be recorded on a recording medium, such as a compact disc read only memory (CD-ROM), a digital versatile disc read only memory (DVD-ROM), or a universal serial bus (USB) memory, and then provided. Further, the soft sensor construction program 70 and the estimation program 80 may be downloaded from an external device through a network.

In addition, the disclosure of JP2021-057497 filed on March 30, 2021 is incorporated herein by reference in its entirety. Further, all documents, patent applications, and technical standards described in the specification are incorporated herein by references to the same extent as the incorporation of the individual documents, patent applications, and technical standards by references are described specifically and individually.

**Claims**

1. A method for estimating a purified state, the method comprising:

   quantifying a component that is included in a treatment liquid obtained by performing a purification treatment on a liquid including a specific protein and impurities other than the protein; and
   acquiring an estimated value of a concentration of the impurities on the basis of spectral data indicating an intensity of electromagnetic waves, which have been emitted to the treatment liquid and have been subjected to an action of the treatment liquid, for each wave number or wavelength,
   wherein the concentration of the impurities included in the treatment liquid is equal to or less than 20 mg/mL, and a weight ratio of the impurities to a mixture including the protein and the impurities is equal to or less than 15%.

2. A method for estimating a purified state, the method comprising:

   quantifying a component that is included in a treatment liquid obtained by performing a purification treatment on a liquid including a specific protein and impurities other than the protein; and
   acquiring an estimated value of a concentration of an immature sugar chain that has a structure similar to that of the protein on the basis of spectral data indicating an intensity of electromagnetic waves, which have been emitted to the treatment liquid and have been subjected to an action of the treatment liquid, for each wave number or wavelength.

3. The estimation method according to claim 1 or 2, further comprising:
   acquiring an estimated value of a concentration of the protein included in the treatment liquid on the basis of the spectral data.

4. The estimation method according to any one of claims 1 to 3,
   wherein the protein is produced from a cultured cell.

5. The estimation method according to any one of claims 1 to 4,
   wherein the impurities include DNA of a cell producing the protein, an aggregate of the protein, a decomposition product of the protein, and a host cell protein.

6. The estimation method according to any one of claims 1 to 5,
   wherein the purification treatment includes a component separation method using chromatography.

7. The estimation method according to any one of claims 1 to 6,
   wherein a determination coefficient indicating a degree of match of the estimated value of the concentration of the impurities with a measured value is equal to or greater than 0.9.

**8.** The estimation method according to any one of claims 1 to 7,
wherein a root mean squared error indicating a degree of deviation of the estimated value of the concentration of the impurities from a measured value is equal to or less than 1.2.

**9.** The estimation method according to any one of claims 1 to 8, further comprising:

constructing a soft sensor, which receives the spectral data as an input and outputs state data indicating a purified state of the liquid including the protein and the impurities, with machine learning using a plurality of combinations of the state data and the spectral data as training data; and
inputting the spectral data acquired for the treatment liquid to the soft sensor and acquiring the state data output from the soft sensor,
wherein the state data includes the estimated value of the concentration of the impurities included in the treatment liquid.

**10.** The estimation method according to claim 9, further comprising:

performing preprocessing on the spectral data; and
constructing the soft sensor with machine learning using a plurality of combinations of processed data obtained by the preprocessing and the state data as training data.

**11.** The estimation method according to claim 10,
wherein the preprocessing includes a process of selecting, from spectral intensity values for each wave number or wavelength included in the spectral data, a spectral intensity value used as the training data.

**12.** The estimation method according to claim 11,
wherein, among the intensities for each wave number or wavelength included in the spectral data, the number of intensities selected to be used as the training data is equal to or greater than 5 and less than 1000.

**13.** The estimation method according to claim 11 or 12,
wherein the selection is performed by sparse modeling.

**14.** The estimation method according to any one of claims 10 to 13,
wherein the preprocessing includes specifying high-correlation spectral data having a relatively high correlation with the state data among the spectral data as the processed data.

**15.** The estimation method according to any one of claims 10 to 14,
wherein the preprocessing includes a baseline correction of the spectral data.

**16.** The estimation method according to any one of claims 9 to 15,
wherein the spectral data is data indicating a spectrum of scattered light of light emitted to the liquid including the protein and the impurities.

**17.** The estimation method according to any one of claims 9 to 16,
wherein the state data includes an estimated value of a concentration of the protein included in the treatment liquid.

# FIG. 1

| PURIFICATION TREATMENT USING IMMUNOAFFINITY CHROMATOGRAPHY | ~P1 |

| VIRUS INACTIVATION TREATMENT | ~P2 |

| PURIFICATION TREATMENT USING CATION CHROMATOGRAPHY | ~P3 |

| PURIFICATION TREATMENT USING ANION CHROMATOGRAPHY | ~P4 |

| VIRUS FILTERING TREATMENT | ~P5 |

| CONCENTRATION AND FILTRATION TREATMENT | ~P6 |

# FIG. 2

20

HIGH-CORRELATION SPECTRAL DATA → SOFT SENSOR → STATE DATA

# FIG. 3

# FIG. 4

50

| No. | SPECTRAL DATA | | | | | STATE DATA CONCENTRATION OF IMPURITIES [g/L] |
|---|---|---|---|---|---|---|
| | 500 cm$^{-1}$ | 501 cm$^{-1}$ | 502 cm$^{-1}$ | ... | 3000 cm$^{-1}$ | |
| 1 | 0.21 | 0.51 | 0.14 | ... | 0.32 | 0.23 |
| 2 | 0.11 | 0.81 | 0.32 | ... | 0.96 | 0.34 |
| ⋮ | ⋮ | ⋮ | ⋮ | ⋮ | ⋮ | ⋮ |

# FIG. 5

20

SPECTRAL INTENSITY VALUE OF 500 cm$^{-1}$
SPECTRAL INTENSITY VALUE OF 501 cm$^{-1}$
SPECTRAL INTENSITY VALUE OF 502 cm$^{-1}$
⋮
SPECTRAL INTENSITY VALUE OF 2650 cm$^{-1}$
SPECTRAL INTENSITY VALUE OF 2651 cm$^{-1}$
SPECTRAL INTENSITY VALUE OF 2652 cm$^{-1}$
⋮
SPECTRAL INTENSITY VALUE OF 3000 cm$^{-1}$

SOFT SENSOR → STATE DATA

# FIG. 6

10

101
CPU

102
MEMORY

104
DISPLAY
UNIT

108

STORAGE UNIT

50 — TRAINING DATA

60 — ESTIMATION
MODEL

70 — SOFT SENSOR
CONSTRUCTION
PROGRAM

80 — ESTIMATION
PROGRAM

INPUT
UNIT

105

NETWORK I/F

106

EXTERNAL I/F

107

103

FIG. 7

FIG. 8

# FIG. 9

START

SELECT SPECTRAL INTENSITY VALUE OF WAVE NUMBER EXCLUDED FROM SPECTRAL DATA ~S1

GENERATE REGRESSION MODEL RELATED TO THINNED-OUT SPECTRAL DATA AND STATE DATA ~S2

DERIVE COST FUNCTION FOR REGRESSION MODEL ~S3

HAS NUMBER OF REPETITIONS REACHED PREDETERMINED NUMBER OF TIMES? ~S4

N

Y

SPECIFY HIGH-CORRELATION SPECTRAL DATA ~S5

CONSTRUCT SOFT SENSOR ~S6

END

# FIG. 10

10

INFORMATION
PROCESSING
DEVICE

SPECTRAL DATA → ACQUISITION UNIT 〜13

EXTRACTION UNIT 〜14

15〜 ESTIMATION UNIT

103

STORAGE UNIT

ESTIMATION MODEL 〜60

# FIG. 11

START

ACQUIRE SPECTRAL DATA 〜S11

EXTRACT HIGH−CORRELATION SPECTRAL DATA 〜S12

ACQUIRE STATE DATA 〜S13

END

## FIG. 12A

RATIO OF IMPURITIES 2.5%

◇ EXAMPLE
■ COMPARATIVE EXAMPLE

$y = 0.9576x - 0.1773$

$y = 0.2552x + 1.2694$

PREDICTION VALUE OF IMPURITIES [mg/mL]

MEASURED VALUE OF CONCENTRATION OF IMPURITIES [mg/mL]

## FIG. 12B

RATIO OF IMPURITIES 5%

◇ EXAMPLE
■ COMPARATIVE EXAMPLE

$y = 0.9327x + 0.254$

$y = 0.4664x + 2.328$

PREDICTION VALUE OF IMPURITIES [mg/mL]

MEASURED VALUE OF IMPURITIES [mg/mL]

## FIG. 12C

RATIO OF IMPURITIES 10%

◇ EXAMPLE
■ COMPARATIVE EXAMPLE

$y = 0.9789x - 0.8281$

$y = 0.3231x + 1.7868$

PREDICTION VALUE OF IMPURITIES [mg/mL]

MEASURED VALUE OF IMPURITIES [mg/mL]

## FIG. 13A

RATIO OF ANTIBODY 20%

◇ EXAMPLE
■ COMPARATIVE EXAMPLE

$y = 1.1052x - 0.4164$

$y = 0.5219x + 0.3084$

PREDICTION VALUE OF CONCENTRATION OF ANTIBODY [mg/mL]

MEASURED VALUE OF CONCENTRATION OF ANTIBODY [mg/mL]

## FIG. 13B

### RATIO OF ANTIBODY 50%

y = 0.9537x − 0.1056

y = 0.6223x + 0.9832

◇ EXAMPLE
■ COMPARATIVE EXAMPLE

PREDICTION VALUE OF CONCENTRATION OF ANTIBODY [mg/mL]

MEASURED VALUE OF CONCENTRATION OF ANTIBODY [mg/mL]

## FIG. 13C

### RATIO OF ANTIBODY 80%

y = 0.9404x − 1.1774

y = 1.044x − 13.035

◇ EXAMPLE
■ COMPARATIVE EXAMPLE

PREDICTION VALUE OF CONCENTRATION OF ANTIBODY [mg/mL]

MEASURED VALUE OF CONCENTRATION OF ANTIBODY [mg/mL]

FIG. 14

## INTERNATIONAL SEARCH REPORT

| International application No. |
| --- |
| **PCT/JP2022/007006** |

### A. CLASSIFICATION OF SUBJECT MATTER

*C07K 1/14*(2006.01)i; *C12Q 1/68*(2018.01)i; *G01N 21/65*(2006.01)i; *G01N 30/88*(2006.01)i
FI: G01N21/65; G01N30/88 J; C07K1/14; C12Q1/68

According to International Patent Classification (IPC) or to both national classification and IPC

### B. FIELDS SEARCHED

Minimum documentation searched (classification system followed by classification symbols)

C07K1/14; C12Q1/00-C12Q3/00; G01N21/00-G01N21/74; G01N30/88; C12M1/00-C12M3/10; C12N1/00-C12N7/08;

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | JP 2019-522802 A (BOEHRINGER INGELHEIM RCV GMBH & CO KG) 15 August 2019 (2019-08-15) paragraphs [0002]-[0004], [0062]-[0172], [0211]-[0239], fig. 1, 8, 11, 12 | 1, 3-15, 17 |
| Y | | 2-17 |
| Y | US 2018/0339244 A1 (KARLSRUHER INSTITUT fur TECHNOLOGIE) 29 November 2018 (2018-11-29) paragraphs [0052]-[0088] | 16-17 |
| Y | WO 2020/160161 A1 (REGENERON PHARMACEUTICALS, INC.) 06 August 2020 (2020-08-06) paragraphs [0128]-[0132], [0182]-[0183] | 16-17 |
| Y | 不完全な糖鎖をきれいに分解するメカニズムを発見, 理化学研究所　研究成果 (プレスリリース), 理化学研究所, 12 Novembger 2013, pp. 1-6, https://www.riken.jp/press/2013/20131112_1/index.html non-official translation (Discovery of mechanism for cleanly decomposing incomplete carbohydrate chain. RIKEN research results (press release), RIKEN.) pp. 2, 3 | 2-17 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **20 April 2022** | **10 May 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2022/007006**

### C. DOCUMENTS CONSIDERED TO BE RELEVANT

| Category* | Citation of document, with indication, where appropriate, of the relevant passages | Relevant to claim No. |
|---|---|---|
| A | KR 10-2005-0044013 A (BIOMND CO.) 12 May 2005 (2005-05-12) entire text, all drawings | 1-17 |
| A | JP 2013-537235 A (ABBVIE INC.) 30 September 2013 (2013-09-30) entire text, all drawings | 1-17 |

Form PCT/ISA/210 (second sheet) (January 2015)

<table>
<tr><td colspan="2">INTERNATIONAL SEARCH REPORT<br>Information on patent family members</td><td colspan="2">International application No.<br><br>**PCT/JP2022/007006**</td></tr>
</table>

| Patent document cited in search report | Publication date (day/month/year) | Patent family member(s) | Publication date (day/month/year) |
|---|---|---|---|
| JP 2019-522802 A | 15 August 2019 | WO 2017/174580 A1<br>paragraphs [0002]-[0004], [0086]-[0195], [0233]-[0256], fig. 1, 8, 11, 12<br>US 2021/0149361 A1<br>EP 3440518 A1<br>CA 3019979 A1<br>AU 2017248025 A<br>SG 11201808401Q A<br>KR 10-2018-0134942 A<br>CN 109313419 A | |
| US 2018/0339244 A1 | 29 November 2018 | WO 2017/088979 A1<br>page 8, line 1 to page 12, line 20<br>EP 3173782 A1 | |
| WO 2020/160161 A1 | 06 August 2020 | JP 2022-519226 A<br>paragraphs [0099]-[0103], [0148]<br>US 2020/0241002 A1 | |
| KR 10-2005-0044013 A | 12 May 2005 | (Family: none) | |
| JP 2013-537235 A | 30 September 2013 | US 2012/0122076 A1<br>entire text, all drawings<br>WO 2012/040041 A1<br>EP 2618904 A1<br>AU 2011305754 A<br>CA 2810909 A1<br>TW 201305187 A<br>CN 103201003 A<br>KR 10-2013-0114143 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200062802 A
- US 20200062802A A

- JP 2021057497 A